# FASCICULE DE BREVET EUROPEEN

(11) **EP 2 118 137 B1**
(45) Date de publication et mention de la délivrance du brevet: **16.03.2011**
(21) Numéro de dépôt: 07872378.0
(22) Date de dépôt: 17.12.2007
(51) Int. Cl.: C07K 14/78, A61K 38/17

(54) **PEPTIDES ET POLYPEPTIDES À STABILITÉ AMÉLIORÉE UTILES DANS LA REGÉNÉRATION DU SYSTÈME NERVEUX**
PEPTIDE UND POLYPEPTIDE MIT VERBESSERTER STABILITÄT, DIE SICH FÜR DIE REGENERATION DES NERVENSYSTEMS EIGNEN
PEPTIDES AND POLYPEPTIDES WITH IMPROVED STABILITY USEFUL IN THE REGENERATION OF THE NERVOUS SYSTEM

(30) Priorité: 18.12.2006 FR 0611005
(43) Date de publication de la demande: 18.11.2009
(73) Titulaire: Neuronax, 63360 Saint-Beauzire (FR)
(72) Inventeur: GOBRON, Stéphane, F-63111 Dallet (FR)
(74) Mandataire: Schmidt, Martin Peter
(86) Numéro de dépôt international: PCT/FR2007/002089
(87) Numéro de publication internationale: WO 2008/090285

(56) Documents cités:
- WO-A-99/03890
- US-A- 5 840 692
- GOBRON S ET AL: "Subcommissural Organ/Reissner's Fiber Complex: Characterization of SCO-Spondin, a Glycoprotein With Potent Activity on Neurite Outgrowth" GLIA, vol. 32, 2000, pages 177-191, XP002442567 cité dans la demande
- GOBRON S ET AL: "SCO-SPONDIN: A NEW MEMBER OF THE TRHROMBOSPONDIN FAMILY SECRETED BY THE SUBCOMMISSURAL ORGAN IS A CANDIDATE IN THE MODULATION OF NEURONAL AGGREGATION" JOURNAL OF CELL SCIENCE, CAMBRIDGE UNIVERSITY PRESS, LONDON, GB, vol. 109, 1996, pages 1053-1061, XP002059006 ISSN: 0021-9533 cité dans la demande
- BAMDAD M ET AL: ".alpha.1.beta.1-Integrin is an essential signal for neurite outgrowth induced by thrombospondin type 1 repeats of SCO-spondin" CELL AND TISSUE RESEARCH, vol. 315, 2004, pages 15-25, XP002442568 cité dans la demande
- MONNERIE H ET AL: "Effect of synthetic peptides derived from SCO-spondin conserved domains on chick cortical and spinal-cord neurons in cell cultures" CELL AND TISSUE RESEARCH, vol. 293, 1998, pages 407-418, XP002442569 cité dans la demande
- EL-BITAR F ET AL: "Effects of SCO-spondin thrombospondin type 1 repeats (TSR) in comparison to Reissner's fiber material on the differentiation of the B104 neuroblastoma cell line" CELL AND TISSUE RESEARCH, vol. 304, 2001, pages 361-369, XP002442570 cité dans la demande

### SEQUENCE LISTING

<110> NEURONAX
<120> Nouveaux peptides et polypeptides à stabilité améliorée utiles dans la régénération du système nerveux
<130> Neuronax
<160> 21
<170> PatentIn version 3.3
<210> 1
   <211> 12
   <212> PRT
   <213> Artificial Sequence
<220>
   <223> Description of Artificial Sequence : Synthetic peptide
<400> 1
   Trp Ser Gly Trp Ser Ser Cys Ser Arg Ser Cys Gly
   1               5                   10
<210> 2
   <211> 10
   <212> PRT
   <213> Artificial Sequence
<220>
   <223> Description of Artificial Sequence : Synthetic peptide
<400> 2
   Trp Ser Gly Trp Ser Ser Cys Ser Arg Ser
   1               5                   10
<210> 3
   <211> 5
   <212> PRT
   <213> Artificial Sequence
<220>
   <223> Description of Artificial Sequence : Synthetic peptide
<400> 3
   Trp Ser Gly Trp Ser
   1               5
<210> 4
   <211> 10
   <212> PRT
   <213> Artificial Sequence
<220>
   <223> Description of Artificial Sequence : Synthetic peptide
<220>
   <221> MOD_RES
   <222> (7) .. (7)
   <223> Abu
<400> 4
   Trp Ser Gly Trp Ser Ser Xaa Ser Arg Ser
   1 5 10
<210> 5
   <211> 10
   <212> PRT
   <213> Artificial Sequence
<220>
   <223> Description of Artificial Sequence : Synthetic peptide
<220>
   <221> MOD_RES
   <222> (10) .. (10)
   <223> Abu
<400> 5
   Trp Ser Gly Trp Ser Ser Cys Ser Arg Xaa
   1 5 10
<210> 6
   <211> 11
   <212> PRT
   <213> Artificial Sequence
<220>
   <223> Description of Artificial Sequence : Synthetic peptide
<220>
   <221> MOD_RES
   <222> (10) .. (10)
   <223> isopropyl-lysine (CH3)2C-NH-(CH2)4-CH(COOH)(NH2)
<400> 6
   Trp Ser Gly Trp Ser Ser Cys Ser Lys Xaa Ser
   1 5 10
<210> 7
   <211> 10
   <212> PRT
   <213> Artificial Sequence
<220>
   <223> Description of Artificiel Sequence : Synthetic peptide
<220>
   <221> MOD_RES
   <222> (2) .. (2)
   <223> Abu
<400> 7
   Trp Xaa Gly Trp Ser Ser Cys Ser Arg Ser
   1 5 10
<210> 8
   <211> 11
   <212> PRT
   <213> Artificial Sequence
<220>
   <223> Description of Artificial Sequence : Synthetic peptide
<220>
   <221> MOD_RES
   <222> (2) .. (2)
   <223> Nla2
<400> 8
   Leu Xaa Ser Gly Trp Ser Ser Cys Ser Arg Ser
   1 5 10
<210> 9
   <211> 10
   <212> PRT
   <213> Artificial Sequence
<220>
   <223> Description of Artificial Sequence : Synthetic peptide
<220>
   <221> MOD_RES
   <222> (7) .. (7)
   <223> Cys or Abu
<400> 9
   Trp Ser Gly Trp Ser Ser Xaa Ser Arg Ser
   1 5 10
<210> 10
   <211> 12
   <212> PRT
   <213> Artificial Sequence
<220>
   <223> Description of Artificial Sequence Synthetic peptide
<220>
   <221> MOD_RES
   <222> (1) .. (3)
   <223> Trip ser, Trip Abu or Leu Na12 ser
<220>
   <221> MOD_RES
   <222> (8) .. (8)
   <223> cys or Abu
<220>
   <221> MOD_RES
   <222> (10) .. (12)
   <223> Arg Ser, Arg Abu or Lys iPr Ser
<400> 10
   Xaa Xaa Xaa Gly Trp Ser Ser Xaa Ser Xaa Xaa Xaa
   1 5 10
<210> 11
   <211> 4
   <212> PRT
   <213> Artficial Sequence
<400> 11
   Val Thr Cys Gly
   1
<210> 12
   <211> 4
   <212> PRT
   <213> Artificial Sequence
<220>
   <223> Description of Artificial Sequence : Synthetic peptide
<400> 12
   Val Ser Cys Gly
   1
<210> 13
   <211> 4
   <212> PRT
   <213> Artificial Sequence
<220>
   <223> Description of Artifïcial Sequence : Synthetic peptide
<400> 13
   Ala Thr Cys Gly
   1
<210> 14
   <211> 4
   <212> PRT
   <213> Artificial Sequence
<220>
   <223> Description of Artificial Sequence : synthetic peptide
<400> 14
   Arg Gly Asp Ala
   1
<210> 15
   <211> 4
   <212> PRT
   <213> Artificial Sequence
<220>
   <223> Description of Artificial Sequence : synthetic peptide
<220>
   <221> MOD_RES
   <222> (1) .. (2)
   <223> Any basic amino acid
<220>
   <221> misc_feature
   <222> (3) .. (3)
   <223> xaa can be any naturally occurring amino acide
<220>
   <221> MOD_RES
   <222> (4) .. (4)
   <223> Any basic amino acid
<400> 15
   Asx Asx Xaa Asx
   1
<210> 16
   <211> 4
   <212> PRT
   <213> Artificial Sequence
<220>
   <223> Description of Artificial Sequence : Synthetic peptide
<220>
   <221> MOD_RES
   <222> (3) .. (3)
   <223> Any amino acid
<400> 16
   Trp Ser Xaa Trp
   1
<210> 17
   <211> 6
   <212> PRT
   <213> Artificial sequence
<220>
   <223> Description of Artificial Sequence : Synthetic peptide
<220>
   <221> MOD_RES
   <222> (3) .. (4)
   <223> Any amino acid
<400> 17
   Cys Ser Xaa Xaa Cys Gly
   1               5
<210> 18
   <211> 5
   <212> PRT
   <213> Artificial Sequence
<220>
   <223> Description of Artificial Sequence : Synthetic peptide
<400> 18
   Trp Ser Pro Trp Ser
   1               5
<210> 19
   <211> 6
   <212> PRT
   <213> Artificiel Sequence
<220>
   <223> Description of Artificial Sequence : Synthetic peptide
<400> 19
   Cys Ser Val Thr Cys Gly
   1               5
<210> 20
   <211> 4
   <212> PRT
   <213> Artificial Sequence
<220>
   <223> Description of Artificial Sequence : Synthetic peptide
<400> 20
   Trp Ser His Trp
   1
<210> 21
   <211> 6
   <212> PRT
   <213> Artificial Sequence
<220>
   <223> Description of Artificial Sequence : Synthetic peptide
<220>
   <221> MOD_RES
   <222> (4) .. (4)
   <223> Any amino acid
<400> 21
   Cys Ser Val Xaa Cys Gly
   1 5

## Description

### Domaine technique de l'invention

La présente invention concerne de nouveaux composés peptidiques à stabilité améliorée dont la séquence dérive de l'un des domaines consensus conservés de la SCO-spondine appelé « répétition thrombospondine de type 1 ou TSR », ainsi que des compositions pharmaceutiques comprenant à titre de principe actif au moins l'un de ces nouveaux composés peptidiques.

### Etat de la technique

Les modulations d'adhésion des cellules entre elles ou des cellules au substrat, ainsi que les phénomènes d'attraction / répulsion, sont des mécanismes importants dans la migration des cellules, et particulièrement lors des étapes de croissance et de guidage des axones pendant le développement du SNC (Tessier-Lavigne et Goodman, 1996; Varela-Echavarria et Guthrie, 1997). La fasciculation des neurites est une étape importante du développement puisqu'elle intervient dans le guidage correct des axones jusqu'à leur cible et sa modulation fait intervenir diverses molécules impliquées dans des phénomènes d'interaction cellule-cellule ou d'adhésion cellulaire comme les molécules d'adhésion cellulaire des nerfs (N-CAMs), les N-cadhérines, les sémaphorines ou des molécules des matrices extracellulaires comme la laminine, la tenascine, les collagènes, la famille des thrombospondines, la fibronectine, la vitronectine, ou des protéoglycanes. Ces différentes molécules peuvent agir en tant qu'inducteurs ou en tant qu'inhibiteurs, le plus souvent par l'intermédiaire d'une liaison ligand / récepteur.

La glycoprotéine SCO-spondine est une protéine spécifique du système nerveux central (SNC) de tous les vertébrés. Elle est synthétisée naturellement par l'organe sous-commissural et fait partie de la fibre de Reissner présente dans le canal central de la moelle épinière. La SCO-spondine fait partie de la superfamille des thrombospondines (voir S. Gobron et al., Journal of Cell Science, vol 109, p. 1053 - 1061 (1996) et J.C. Adams et al., Developmental Dynamics, vol. 218, p. 280 - 29 (2000)), Les thrombospondines sont produites par de nombreuses cellules saines. Elles comprennent trois chaînes de polypeptides reliées par deux ponts di-sulfide.

La SCO-spondine possède des homologies avec des membres de cette superfamille des thrombospondines, dont certains sont aussi exprimés au niveau du SNC : les thrombospondines 1 et 2, certaines sémaphorines, les mindines ou la F-spondine par exemples (S. Gobron et al., Glia, vol 32, p. 177-191 (2000)). La séquence de toutes ces molécules comprend des motifs protéiques consensus appelés répétitions thrombospondine de type 1 (TSR).

La SCO-spondine comprend environ 4500 acides aminés (4560 selon S. Gobron et al., Glia, vol 32, p. 177-191 (2000)), et sa structure montre un arrangement unique de plusieurs domaines conservés incluant vingt-six motifs TSR, neuf domaines de récepteurs de type A de lipoprotéines de faible densité (LDL-R), deux domaines de facteurs de croissance de l'épiderme (EGF), et, des domaines N-terminal et C-terminal du facteur von Willebrand (vWF) riches en cystéine qui sont tous des sites potentiels d'interactions protéine-protéine (S. Gobron et al., Glia, vol 32, p. 177-191 (2000)). La structure de la glycoprotéine SCO-spondine a été décrite par exemple par A. Meiniel (Microscopic Research and Technique, vol 52, p. 484-495 (2001)).

Les thrombospondines sont des composants des matrices extracellulaires qui sont impliqués dans un grand nombre de processus physiologiques et qui présentent, d'une façon générale, des propriétés adhésives ou anti-adhésives *in vitro,* suggérant leur rôle sur le comportement des cellules *in vivo.* En effet, différentes parties de la molécule sont suspectées d'interagir avec d'autres molécules des matrices extracellulaires ainsi qu'avec différents récepteurs cellulaires modulant ainsi les contacts cellule-cellule (J. C. Adams et al., Molecular Biology Intelligence Unit - ed. R.G. Landes, Company - Springer Verlag, p. 1-188 (1995)). L'attachement direct de la thrombospondine-1 à différentes cellules de divers tissus tels que les cellules hématopoïetiques, les cellules neuronales, les cellules épithéliales, a également été montré ainsi que des modifications de son expression dans diverses pathologies, en particulier des cancers, et dans des processus de cicatrisation (Adams et al., 1995, cité ci-dessus, pour revue). De plus, différents types de neurones cultivés in vitro en présence de thrombospondine 1 ont montré des extensions neuritiques accrues; ce phénomène pouvant être lié à la présence d'une portion de la molécule comprenant des motifs protéiques particuliers appelés « répétitions thrombospondine de type 1 » ou TSR (K. O'Shea et al., Neuron, vol. 7 p. 231-237 (1991)):

En fait, la thrombospondine comporte entre autres trois de ces motifs TSR. La fonction de chacun d'entre eux à été bien étudiée (pour revue voir : H. Chen et al. Matrix Biology, vol. 19 p. 597-614 (2000)) et la cristallisation de ces motifs TSR a apporté des éclairages supplémentaires (K. Tan, The Journal of Cell Biology, vol. 159 (2), 373-3.82 (2002)). Par exemple, il a été montré que des peptides provenant de trois motifs TSR se lient à des composés glycoconjugués sulfatés incluant l'héparine ou la sulfatide (US 5 357 041) et se sont révélés utiles dans des compositions pharmaceutiques pour lier des composés glycoconjugués.

Les motifs TSR sont des domaines protéiques d'environ 55 résidus, basés sur l'alignement d'acides aminés conservés cystéine, tryptophane et arginine. "
Ces motifs ont tout d'abord été isolés dans la thrombospondine 1 qui est une molécule intervenant dans la coagulation, puis ont ensuite été décrits dans de nombreuses autres molécules aux fonctions biologiques diverses telles que l'attachement cellulaire, la mobilité, la prolifération, l'agrégation cellulaire, la modulation de protéases ou l'inhibition de l'angiogénèse (Adams et al., 1995, pour revue).

Ainsi, ces motifs ont été observés dans :
- la F-spondine, molécule sécrétée pendant le développement embryonnaire par les cellules de la lame basale du tube nerveux, ou "floor plate" (A. Klar et al., Cell, vol. 69, p. 95-110 1992), et impliquée dans des phénomènes d'adhésion cellulaire et de pousse neuritique par l'intermédiaire de différentes régions de la protéine (Y. Feinstein et A. Klar, The International Journal of Biochemistry & Cell Biology, vol. 36 p. 975-980 2004, D. Schubert, A. Klar et al., Journal of Neurochemistry, vol. 96, p. 444-453 2006, pour revue);
- les sémaphorines F et G, membres d'une famille de protéines sécrétées dans le milieu extracellulaire et membranaires, impliquées dans des phénomènes de guidage des axones, mais dont la fonction exacte n'est pas bien connue (A. Varela-Echavarria et S. Guthrie, Gene & Development, vol. 11 p. 545-557 1997, pour revue).

Ces motifs protéiques ont également été identifiés dans d'autres molécules exprimées en dehors du SNC, telles que la properdine, qui est une protéine de liaison du système du complément et qui possède six TSRs (D. Goundis et K. Reid, Nature, vol. 335 p. 82-85 1988).

Ces motifs TRS sont retrouvés dans différents organismes.

Par exemple, dans la protéine CS, protéine exprimée par le *plasmodium,* sporozoïte vecteur de la malaria (K. Robson et al., Nature, vol. 335 p. 79-82 1988), il a été montré que la présence de ce motif TSR joue un rôle primordial dans l'étape, de liaison du sporozoïte à la cellule hôte (Tewari R. et al., The Journal of Biological Chemistry, vol. 277, p. 47613-47618,2002).

Aussi, dans une molécule intervenant dans la croissance axonale, chez le nématode C. *elegans,* appelée Unc-5 (E. Hedgecock et al., Neuron, vol. 2, p. 61-85 (1990)), et pour laquelle la mutation de son gène conduit à des défauts dans le guidage des axones chez *C. elegans* (C. Leung-Hagesteijn et al., Cell, vol. 71, p. 289-299 1992).

Par ailleurs, il a été montré que la thrombospondine est particulièrement abondante dans les faisceaux de fibres en développement et dans les régions de croissance des axones, suggérant ainsi un rôle dans l'établissement d'un environnement favorable permettant la croissance des axones (K. S. O'Shea et V. M. Dixit, The Journal of Cell Biology, vol. 107 p. 2737-2748 (1988)).

Dans la throinbospondine, d'une façon générale, il a été montré que les motifs TSR peuvent être impliqués dans les mécanismes de modulation de l'adhésivité cellulaire ; ce phénomène peut jouer un rôle lors de la pousse neuritique et du guidage axonal.

En ce qui concerne l'effet de la thrombospondine 1 sur l'induction des extensions neuritiques, l'intervention de récepteurs spécifiques présents à la surface des cellules nerveuses a été suggérée. Ainsi, des anticorps dirigés spécifiquement contre l'intégrine α3β1 ont permis d'inhiber la croissance neuritique de neurones sympathiques cultivés sur la thrombospondine-1 (M. De Freitas et al., Neuron, vol. 15 p. 333-343 (1995)).

On sait que d'une manière générale certains fragments peptidiques de la thrombospondine, ainsi que certains peptides synthétiques dont la structure est analogue à celle de ces fragments peptidiques, montrent une activité biologique similaire à celle de la trombospondine. A titre d'exemples, les brevets US 6,239,110 (W.R. Grace & Co. - Conn. et Medical College of PA), US 5,840,692 (Allegheny University of the Health Sciences et W.R. Grace, Co. - Conn.), US 5,849,701 (The United States of America), US 5,491,130 et US 6,051,549 (The United States of America), US 5,190,918 et US 5,200,397 (W.R. Grace & Co. - Conn. et The Medical College of Pennsylvania), et US 6,384,189 décrivent la structure et les effets biologiques de plusieurs de ces fragments peptidiques déduits de la thrombospondine et de ses analogues synthétiques.

Mais on sait aussi que des peptides déduits spécifiquement de la SCO-spondine présentent une activité biologique sur les cellules nerveuses (demande de brevet WO 99/03890), et que la SCO-spondine agit sur les cellules par l'intermédiaire d'un récepteur cellulaire spécifique qui diffère de ceux décrits précédemment pour la thrombospondine (M. Bamdad et el., Cell & Tissue Research, vol. 315, 15-25 (2004)).

Les caractéristiques détaillées de ces motifs TSR sont maintenant bien connues.

Ainsi, des propriétés de liaison cellulaire, de liaison à l'héparine, à la fibronectine ou au facteur de croissance transformant β (TGFβ) ont été rapportées pour certaines séquences spécifiquement présentes à l'intérieur du domaine TSR (Adams et al., 1995, pour revue).

Les propriétés d'adhésion conférées par la séquence peptidique V-T-C-G (SEQ ID N°11) ont également été montrées dans le cas de la protéine CS du *plasmodium* (K. Rich et al., Science, vol. 249 p. 1574-1577 1990), ou ses variantes, V-S-C-G (SEQ ID N°12) et A-T-C-G (SEQ ID N°13), dans la fonction biologique de la properdine (C. Prater et al., The Journal of Cell Biology, vol. 112 p. 1031-1040 1991).

La séquence V-T-C-G (SEQ ID N°11) de la thrombospondine peut se lier au récepteur CD 36 ou gpIV des plaquettes.

La séquence R-G-D-A (SEQ ID N°14) peut se lier à l'intégrine αVβ3 et le domaine carboxy terminal peut se lier au récepteur hétérodimérique 105/80 kDa.

La séquence B-B-X-B (SEQ ID N°15) (où B est un acide-aminé basique et X n'importe quel acide aminé) peut se lier aux protéoglycanes (W. A. Frazier, Current Opinion in Cell Biology, vol. 3, P. 792-799 1991, Adams et al., (1995)).

On sait que les motifs W-S-X-W (SEQ ID N°16) C-S-X-X-C-G (SEQ ID N°17) ou V-T-C-G (SEQ ID N°11) ont chacun des activités biologiques distinctes.

Le motif W-S-X-W (SEQ ID N°16) a été montré comme capable de lier l'héparine est des protéoglycanes permettant ainsi un effet biologique variable suivant le type de cellules Ce motif a également été montré comme intervenant dans le processus d'activation du TGFβ par la thrombospindine-1. Bien que cette activité ait été attribuée à une région située en amont de ce motif dans le domaine TSR, la présence de ce motif W-S-X-W (SEQ ID N°16) semble nécessaire, mais non suffisant à lui seul pour cette activation de la cytokine, qui pourra alors intervenir dans différent événements biologiques tels que l'inflammation, l'angiogénèse ou le développement embryonnaire (G. D. Young et J. E. Murphy-Ullrich, The Journal of Biological Chemistry, vol. 279, p. 47633-47642 (2004)). Cette propriété de W-S-X-W (SEQ ID N°16) à avoir une affinité pour l'héparine ou les protéoglycanes semble également intervenir dans les propriétés anti-angiogéniques de la thrombospondine-1, puisque ce motif semble agir comme compétiteur de certains facteurs de croissance pro-angiogéniques lors de leur liaison aux protéoglycanes présents à la surface des cellules endothéliales (T. Vogel et al., The Journal of Cellular Biochemistry, vol. 53 p. 74-84 (1993)). En effet, une des activités des thrombospondines particulièrement étudiée est sa capacité à inhiber la néovascularisation. Différentes études ont cherché à identifier la région de la molécule qui était responsable de cette activité (pour revue voir M.L. Iruela-Arispe, The International Journal of Biochemistry & Cell Biology, vol 36 p.1070-1078(2004)).

A titre d'exemple, on peut citer S.S. Tolsma et al. (The Journal of Cell Biology, vol. 122 p.497-511 (1993)) qui ont montré que l'activité antiangiogénique de la thrombospondine-1 était localisée dans deux régions distinctes. Ainsi, l'activité de la molécule pouvait être reproduite *in vitro* et *in vivo* avec différents peptides synthétiques, dont en particulier deux séquences déduites du domaine TSR et qui présentent en position centrale le motif C-S-V-T-C-G (SEQ ID N°19).

Des études ultérieures ont toutefois précisé que cette activité d'inhibition de la néovascularisation par la thrombospondine-1 pouvait se dérouler selon deux mécanismes différents qui faisaient intervenir deux régions différentes du TSR :
- une région renfermant le motif W-S-X-W (SEQ ID N°16), et
- une autre région renfermant le motif C-S-V-T-C-G (SEQ ID N°19) (M.L. Iruela-Arispe, Circulation, vol. 100 p.1423-31 (1999)).

On sait également que dans le cas de peptides comportant le motif W-S-X-W (SEQ ID N°16), la constitution exacte de l'acide aminé variant (X) ou des acides aminés additionnels situés en amont ou en aval de ce motif est importante pour l'activité du peptide. C'est le cas en particulier de l'efficacité de la liaison à l'héparine et de l'induction de l'adhésion cellulaire qui se sont trouvées augmentées pour un pentapeptide où X était la proline (P) ou des peptides plus grands comportant des acides aminés basiques autour du motif W-S-X-W (SEQ ID N°16) (N.H. Guo, The Journal of Biological Chemistry, vol. 267 p.19349-55 (1992)).

C'est le cas également de la liaison à la fibronectine qui est plus importante pour les peptides comportant deux résidus glycine (G) en amont du motif W-S-H-W (SEQ ID N°20) (J.M. Sipes, The Journal of Cell Biology, vol. 121 p.469-477 (1993)).

Ainsi, les capacités des domaines TSR à intervenir sur les phénomènes d'adhésion cellulaire seraient essentiellement dues à la présence du motif W-S-X-W (SEQ ID N°16) et non à C-S-X-X-C-G (SEQ ID N°17).

Néanmoins, des résultats ont été obtenus concernant l'implication de ce dernier motif, C-S-X-X-C-G (SEQ ID N°17), dans la liaison des TSR aux cellules. Ainsi, Tuszynski et al. ont confirmé des travaux antérieurs, en montrant que C-S-V-T-C-G (SEQ ID N°19) et des dérivés étaient importants pour l'interaction dans des phénomènes d'adhésion cellulaire (The Journal of Cell Biology, vol. 116 p.209-217 (1992)), alors que Gantt et al. ont montré que des acides aminés basiques situés en aval du motif C-S-V-T-C-G (SEQ ID N°19), un variant de C-S-X-X-C-G (SEQ ID N°17) étaient nécessaires à la liaison directe du domaine TSR aux cellules (S.M. Gantt, The Journal of Biological Chemistry, vol. 272 p. 19205-19213 (1997)). Il est à noter que ces derniers auteurs suggèrent également que le processus d'adhésion des molécules de thrombospondine par les domaines TSR se déroulerait en plusieurs étapes dont :
- la première consisterait en une liaison de W-S-X-W (SEQ ID N°16) aux protéoglycanes présents à la surface cellulaire, et
- une deuxième consisterait en la reconnaissance par C-S-X-X-C-G (SEQ ID N°17) d'un récepteur cellulaire spécifique.

De plus, dans d'autres systèmes cellulaires :
- le peptide W-S-P-W-S (SEQ ID N°18) peut influencer la croissance neuritique de neurones embryonnaires de rat *in vitro* (D. Osterhout et al., Annual Meeting - Washington (1993)),
- le peptide C-S-V-T-C-G (SEQ ID N°19) a clairement été montré comme responsable du phénomène de liaison cellulaire de la protéine CS du *plasmodium* aux cellules hématopoïetiques (K.A. Rich, Science, vol. 249 p.1574-1577 (1990)), la région de la molécule comportant ce motif ayant même était considérée comme ayant un rôle critique dans l'infectivité du *plasmodium in vivo* (K. Tewari, The Journal of Biological Chemistry, vol. 277p. 47613-47618 (2002)). On peut en déduire que la présence du motif C-S-X-X-C-G (SEQ ID N°17) serait bien nécessaire à la fixation et à l'activité du domaine TSR qui en découle.

On sait également que toutes les activités biologiques des thrombospondines, et de la thrombospondine 1 en particulier, ne sont pas seulement dues à la présence de ces domaines TSR puisque d'autres régions, et en particulier la région carboxy-terminale de cette protéine, comportent des motifs de nature différente, qui sont également impliquées dans les différents phénomènes d'adhésion, migration ou prolifération cellulaire (voir pour revue J. C. Adams, The International Journal of Biochemistry & Cell Biology, vol. 36 p. 1102-1114 (2004)). Il en va de même pour les autres molécules comportant les motifs TSR pour lesquelles les activités ont finalement été reliées à d'autres domaines fonctionnels différents des TSR. Ainsi, par exemple, la F-spondine a tout d'abord montré des capacités à induire la croissance neuritique *in vitro,* capacités qui ont été suggérées par la caractérisation de motifs TSR dans sa séquence (Klar et al 1992). Toutefois, des expérimentations ultérieures ont montré que des anticorps dirigés spécifiquement contre des domaines TSRs de la F-spondine ne bloquaient pas l'activité d'induction de la pousse neuritique observée lorsque des neurones sont cultivés en présence de F-spondine, alors que des anticorps dirigés contre un autre domaine de cette molécule et appelé « spondiné » étaient quant à eux bloquants (T. Burstyn-Cohen et al., The Journal of Neuroscience, vol. 18 p.8875-8885 (1998)), suggérant ainsi que la présence de ces motifs TSRs dans une molécule ne signifiait pas forcément une activité d'induction de pousse neuritique.

C'est le cas également d'Unc-5, pour laquelle le rôle exact des domaines TSRs n'est pas clairement établi, bien qu'ils apparaissent importants pour la fonction de la protéine native *in vitro* et *in vivo* (R.P. Kruger, The Journal of Neuroscience, vol. 24 p. 10826-10834 (2004)).

Les motifs TSR étant très nombreux dans la séquence de la SCO-spondine, l'activité biologique d'un peptide de synthèse dont la séquence en acides aminés :
W-S-G-W-S-S-C-S-R-S-C-G (SEQ ID N°1),
correspond à la séquence la plus représentative d'un motif TSR de la SCO-spondine, a été testée sur des modèles de cultures de cellules nerveuses, à savoir des cellules B104, issues d'un neuroblastome de rat. Les résultats obtenus montrent que le peptide W-S-G-W-S-S-C-S-R-S-C-G (SEQ ID N°1) entraîne une différenciation cellulaire : il induit la pousse neuritique et l'agrégation cellulaire (voir H. Monnerie et al., Cell Tissue Res., vol. 293, p. 407 - 418 (1998) ; voir F. El-Bitar et al., Cell Tissue Res., vol. 304, p. 361-369 (2001) ; voir la demande de brevet WO 99/03890 (Université d'Auvergne) et notamment la séquence SEQ ID N° 8 de ce document).

En particulier, il à été démontré par Gobron et al. (Glia, vol. 32, p. 177-191 (2000)) que pour le modèle choisi, l'effet du peptide W-S-G-W-S-S-C-S-R-S-C-G (SEQ ID N°1) sur la pousse neuritique est dépendant de la dose de peptide utilisée. Dans ces expériences, les cellules de la lignée B104 ont été ensemencées dans des puits de culture, puis mises en contact avec le peptide W-S-G-W-S-S-C-S-R-S-C-G (SEQ ID N°1) dans une gamme croissante de concentrations de peptide.

Après 18 heures de culture, en l'absence de sérum, les cellules ont commencé à esquisser un ou deux prolongements neuronaux, et ceci indépendamment de la présence ou de l'absence de peptide.

Après trois jours de culture, des pousses neuritiques importantes ont été observées chez les cellules traitées par le peptide W-S-G-W-S-S-C-S-R-S-C-G (SEQ ID N°1), alors que dans les cultures témoins (sans peptide), les cellules ne présentaient pratiquement pas d'excroissance neuritique.

Les auteurs ont constaté que cette réponse des cellules B104 était proportionnelle à la concentration de peptide ajoutée au milieu. En effet, le nombre de neurites et la taille du neurite le plus long par cellule augmentait progressivement en suivant les concentrations croissantes de peptide.

L'observation morphologique de la pousse neuritique et de l'agrégation cellulaire décrite par F. El-Bitar et al. (Cell and Tissue Research vol. 304, 361-369 (2001)) a montré que le peptide W-S-G-W-S-S-C-S-R-S-C-G (SEQ ID N°1) induit une augmentation :
- du nombre de neurites, ainsi que
- de la taille du neurite le plus long,
tandis que dans les cultures témoins, aucun changement cellulaire n'a été observé.

En effet, durant les premières vingt-quatre heures, les cellules B 104 sont restées étalées dans les cultures traitées et témoins.

Dans les puits traités avec le peptide W-S-G-W-S-S-C-S-R-S-C-G (SEQ ID N°1), les auteurs ont remarqué, au bout de 2 jours de culture, que les cellules présentaient d'importantes extensions neuritiques. Ces extensions étaient encore plus marquées au 3^{ième} jour de culture Alors que dans les cultures témoins, les cellules ne montraient que peu de modifications morphologiques.

A partir de 3 ou 4 jours de culture en présence du peptide W-S-G-W-S-S-C-S-R-S-C-G (SEQ ID N°1), les auteurs ont pu observer l'apparition de regroupements de ces cellules B 104 différenciées, et il n'était plus possible de distinguer les neurites propres à chaque cellule. Puis, ces regroupements cellulaires se sont accentués à 5 jours de culture, et donnaient naissance à de petits amas denses, reliés entre eux par des faisceaux neuritiques, après le 6^{ième} jour de culture.

Dans les cultures témoins, aucun regroupement cellulaire n'est apparu, les cellules constituant un tapis cellulaire homogène au fond des puits.

Il apparaît donc que le peptide W-S-G-W-S-S-C-S-R-S-C-G (SED ID N°1) présente des activités biologiques intéressantes sur les cellules du système nerveux, ce qui en fait un bon candidat-médicament pour le traitement de certaines pathologies du système nerveux.

Il a été montré que le peptide W-S-G-W-S-S-C-S-R-S-C-G (SEQ ID N°1) agissait par l'intermédiaire d'un récepteur spécifique présent à la surface des cellules, celui-ci activant une cascade intracellulaire aboutissant à des modifications morphologiques, dont en particulier l'induction de la pousse neuritique. Cette cascade fait intervenir, en fonction du temps, une cinétique de différents partenaires moléculaires successifs (tels que phosphorylases, constituants des microfilaments) aboutissant finalement à la formation des extensions nerveuses.

Cet effet du peptide W-S-G-W-S-S-C-S-R-S-C-G (SEQ ID N°1) a également été montré comme dépendant de la dose employée : une dose optimale pouvant être caractérisée selon le système cellulaire (cellules nerveuses primaires ou cellules issues de lignées) utilisé. Toutefois, il est apparu qu'une optimisation de l'effet de ce peptide pouvait être obtenue lorsque celui-ci était délivré régulièrement à la cellule cible. Ainsi, il est apparu que lorsque ce peptide est ajouté quotidiennement au milieu de culture de cellules nerveuses à sa dose optimale, l'effet observé était accentué.

Cependant, le présent inventeur a trouvé que la stabilité du peptide W-S-G-W-S-S-C-S-R-S-C-G (SEQ ID N°1), constitué de 12 acides aminés naturels, diminue en solution aqueuse avec le temps. Il est donc possible que le peptide devra être préparé extemporanément aux traitements.

Dans certaines situations, cela pourrait compliquer l'administration du médicament aux patients, et d'une manière générale, présenter un certain inconvénient pour le développement de concepts thérapeutiques basés sur ce peptide.

La présente invention vise à améliorer la stabilité du peptide W-S-G-W-S-S-C-S-R-S-C-G (SEQ ID N°1) en solution aqueuse.

### Description des figures

La figure 1 montre la pureté des peptides en % déterminée par HPLC après une durée de stockage déterminée à 22°C.
La figure 2 montre un résultat comparatif de l'activité biologique de trois peptides :
   - Peptide A = le peptide de séquence SEQ ID N°2 qui se présente sous la forme : W-S-G-W-S-S-C-S-R-S-NH₂;
   - Peptide TSR n°1 = le peptide de séquence SEQ ID N°1 qui se présente sous la forme : W-S-G-W-S-S-C-S-R-S-C-G-OH;
   - Peptide TSR n°2 = le peptide de séquence SEQ ID N°1 qui se présente sous la forme : W-S-G-W-S-S-C-S-R-S-C-G-NH₂.

Les concentrations de peptide sont exprimées pour un volume total de 200 µL par puits. Le Témoin étant une culture de cellules en milieu dépourvu de sérum.

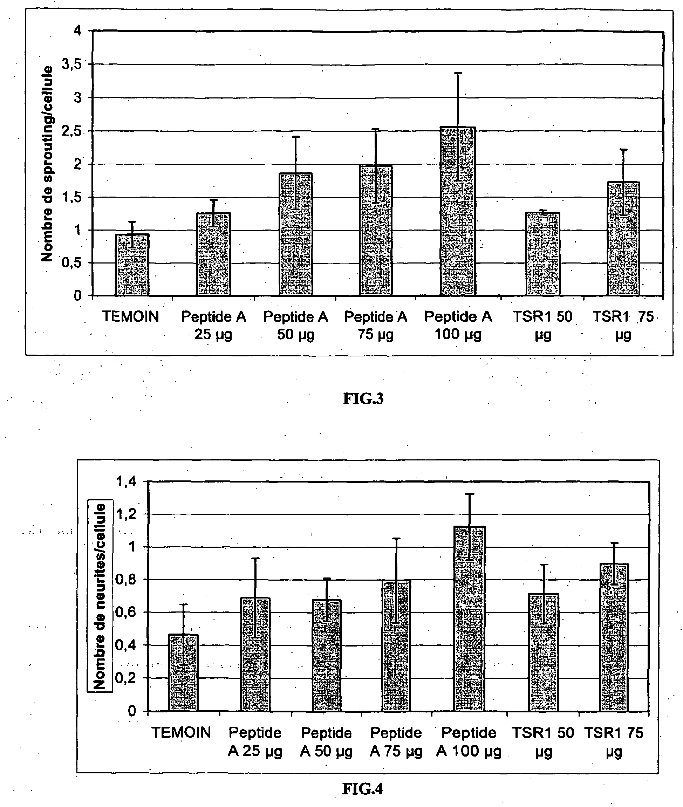

La figure 3 montre un résultat comparatif du nombre de sprouting par cellule après 3 jours de culture.

La figure 4 montre un résultat comparatif du nombre de neurites par cellule après 3 jours de culture.

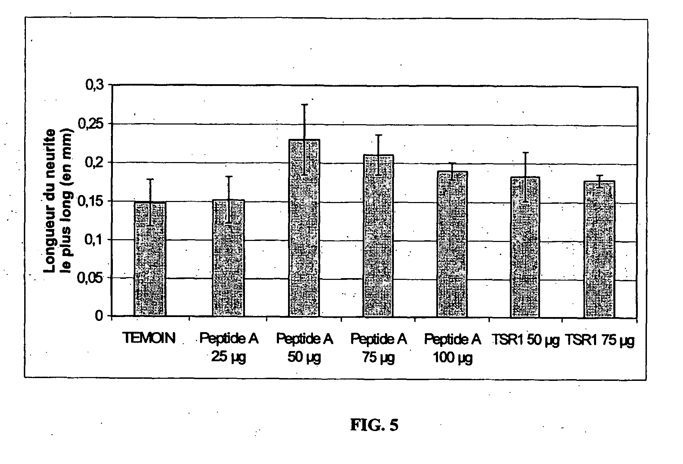

La figure 5 montre la longueur de la pousse neuritique après 3 jours.

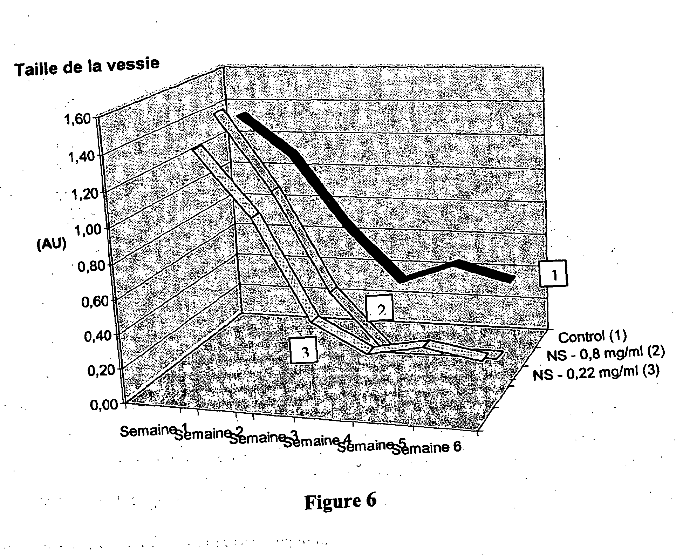

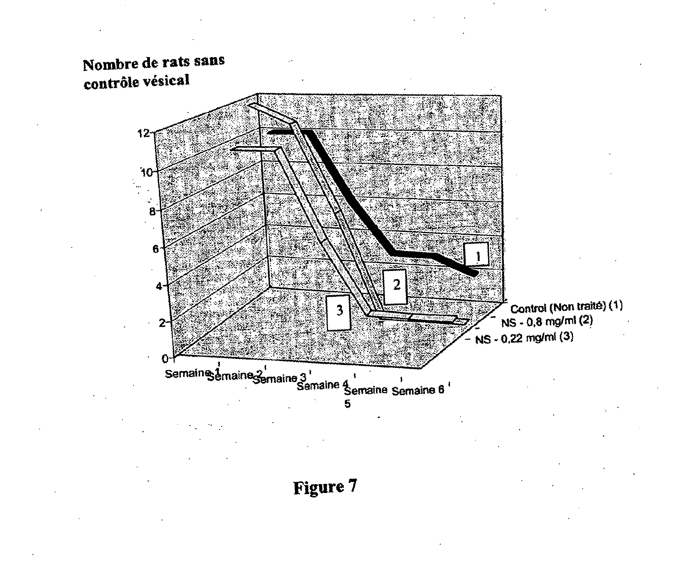

Les figures 6 et 7 montrent les résultats obtenu avec le composé peptidiques SEQ ID N° 4, pour un groupe de contrôle de rats ayant subi une lésion de la moelle épinière, sans traitement (courbe 1), et avec une concentration de la peptide à 0,8 m/ml (courbe 2) ou 0,22 mg/ml (courbe 3). La figure 7 montre le nombre de rats qui étaient après 1, 2, 3, 4, 5 ou 6 semaines sans contrôle vésical. La figure 6 montre, en unités arbitraires, la taille de la vessie après 1, 2, 3, 4, 5 ou 6 semaines.

Dans les figures 3, 4 et 5, les concentrations de peptide sont exprimées pour un volume total de 200 µL par puits. Le témoin représente une culture de cellules en milieu dépourvu de sérum.

### Objet de l'invention

Un premier objet de la présente invention est un composé peptidique contenant au moins la séquence en acides aminés suivante :
X2-G-W-S-S-X1-S-X3 (SEQ IDN°10)
dans lequel :
X1 est C ou Abu ;
X2 est W-S ou W-Abu ou L-Nal2-S ;
X3 est R-S, R-Abu ou K-(iPr)-S ;
ledit composé étant caractérisé en ce que la séquence (SEQ ID N°10) est sélectionnée dans le groupe constitué par les séquences :
W-S-G-W-S-S-Abu-S-R-S (SEQIDN°4)
W-S-G-W-S-S-C-S-R-Abu (SEQ ID N°5)
W-S-G-W-S-S-C-S-K-(iPr)-S (SEQ ID N°6)
W-Abu-G-W-S-S-C-S-R-S (SEQIDN°7)
L-Nal2-S-G-W-S-S-C-S-R-S (SEQ ID N°8).

Un second objet de l'invention est une composition pharmaceutique comprenant à titre de principe actif au moins un des composés peptidiques indiqués ci-dessus comme premier objet.

Un troisième objet de l'invention est l'utilisation, et notamment l'utilisation extracorporelle, des composé peptidiques indiqués ci-dessus comme premier objet dans la régénération des cellules du système nerveux.

Un quatrième objet de l'invention est l'utilisation des composés peptidiques indiqués ci-dessus pour la préparation de médicaments, et notamment pour la réalisation de médicaments, destinés au traitement des maladies neurodégénératives et / ou à la régénération de la moelle épinière ou des nerfs périphériques après traumatismes.

Un cinquième objet de l'invention est un additif pour les cultures cellulaires de cellules nerveuses, caractérisé en ce qu'il comporte au moins un des composés peptidiques indiqués ci-dessus comme premier objet.

Cet additif peut être utilisé dans un support pour la culture de cellules nerveuses, qui constitue le sixième objet de l'invention.

### Description de l'invention

### 1. Définitions

Dans le cadre de la présente invention, nous entendons par « acide aminé» : les acides aminés naturels et les acides aminés non naturels.

Les acides aminés « naturels » comprennent la forme L des acides aminés qui peuvent être trouvés dans des protéines d'origine naturelle, c'est-à-dire : alanine (A), arginine (R), asparagine (N), acide aspartique (D), cystéine (C), glutamine (Q), acide glutamique (E), glycine (G), histidine (H), isoleucine (I), leucine (L), lysine (K), méthionine (M), phénylalanine (F), proline (P), sérine (S), thréonine (T), tryptophane (W), tyrosine (Y) et valine (V).

Les acides aminés « non naturels » comprennent la forme D des acides aminés naturels, les formes *homo* de certains acides aminés naturels (tels que: arginine, lysine, phénylalanine et sérine), et les formes nor de la leucine et de la valine. Is comprennent aussi des acides aminés synthétiques, tels que :
- Abu =: acide 2-aminobutyrique CH₃-CH₂-CH(GOOH)(NH₂)
- iPr =: isopropyl-lysine (CH₃)₂C-NH-(CH₂)₄-CH(COOH)(NH₂)
- Aib =: acide 2-aminoisobutyrique
- F-trp =: N-formyl-tryptophane
- Orn =: ornithine
- Nal2 =: 2-naphthylalanine

On entend par « poussée neuritique » : l'allongement, c'est-à-dire la croissance des prolongements des neurones, que ce soit le prolongement axonal ou dendritique.

On entend par « agrégation » : le regroupement des cellules formant un amas.

On entend par « défasciculation » : le résultat d'une diminution de l'adhésivité entre neurites, conduisant à un réseau lâche de prolongements neuronaux.

Les « composés peptidiques » comprennent notamment les peptides et les polypeptides.

### 2. Description détaillée de l'invention

L'inventeur a trouvé qu'une modification de la séquence du peptide W-S-G-W-S-S-C-S-R-S-C-G (SEQ ID N°1) peut conduire à l'amélioration de sa stabilité, tout en gardant une activité biologique comparable ou même supérieure comparée à celle du peptide de départ. Plus précisément, la séquence :
X2-G-W-S-S-X1-S-X3 (SEQ ID N°10)
dans laquelle :
- X1 signifie soit C soit Abu, et
- X2 signifie soit W-S, soit W-Abu, soit encore L-Nal2-S, et
- X3 signifie R-S, R-Abu ou K=(iPr)-S, ledit composé étant caractérisé en ce que la séquence (SEQ ID N°10) est sélectionnée dans le groupe constitué par les séquences :
   W-S-G-W-S-S-Abu-S-R-S (SEQIDN°4)
   W-S-G-W-S-S-C-S-R-Abu (SEQ ID N°5)
   W-S-G-W-S-S-C-S-K-(iPr)-S (SEQ ID N°6)
   W-Abu-G-W-S-S-C-S-R-S (SEQ ID N°7)
   L-Nal2-S-G-W-S-S-C-S-R-S (SEQ ID N°8),
montre par rapport à la séquence initiale (SEQ ID N°1) à la fois :
- une stabilité en solution améliorée, et
- une bonne activité biologique.

### 2.1 Design de nouveaux peptides

Sur la base de l'idée que l'instabilité du peptide W-S-G-W-S-S-C-S-R-S-C-G (SEQ ID N°1) pourrait être due à un changement de conformation, et en particulier liée aux interactions entre les deux unités de cystéine de la séquence, l'inventeur a tout d'abord conçu deux premiers peptides A et B :
- Le peptide A a la séquence linéaire :
   W-S-G-W-S-S-C-S-R-S (SEQ ID N°2),
      dans laquelle par rapport à la séquence initiale W-S-G-W-S-S-C-S-R-S-C-G (SEQ ID N°1), les deux derniers acides aminés cystéine et glycine sont supprimés.

   Il a été préparé selon les standards habituels sous la forme :
   _{NH2}-W-S-G-W-S-S-C-S-R-S-_{COOH.}
- Le peptide B a la séquence linéaire :
   W-S-G-W-S (SEQ ID N°3),
      qui correspond à un motif protéique consensus décrit dans la littérature mais qui ne semble pas intervenir dans des phénomènes de pousse neuritique.

Il a été préparé sous la forme _{NH2}-W-S-G-W-S-_{COOH}.

Il est à noter que le peptide A de séquence W-S-G-W-S-S-C-S-R-S (SEQ ID N°2) ne comporte plus le motif C-S-V-X-C-G (SEQ ID N°21) abondamment décrit comme intervenant dans des phénomènes d'adhésion cellulaire (Exemple malaria).

Deux manipulations concernant le peptide A ont également été effectuées et on montré des résultats significatifs.

Elles ont en effet montré que ce peptide A, bien qu'étant tronqué de ces deux acides-aminés terminaux cystéine et glycine impliqués dans le motif consensus C-S-V-X-C-G (SEQ ID N°21), a conservé son activité biologique.

Il semble donc que l'effet observé initialement par le peptide de séquence W-S-G-W-S-S-C-S-R-S-C-G (SEQ ID N°1) fasse intervenir un mécanisme différent de celui classiquement décrit pour les molécules présentant des motifs de ce type et dans lesquelles le motif C-S-V-X-C-G (SEQ ID N°21) a été décrit comme responsable de l'activité biologique.

Puis, afin de comparer la stabilité des différents peptides dérivés du peptide W-S-G-W-S-S-C-S-R-S-C-G (SEQ ID N°1), d'autres peptides de la séquence :
X2-G-W-S-S-X1-S-X3 (SEQ ID N°10)
ont été préparés :
W-S-G-W-S-S-Abu-S-R-S (SEQ ID N°4)
W-S-G-W-S-S-C-S-R-Abu (SEQ ID N°5)
W-S-G-W-S-S-C-S-K-(iPr)-S (SEQ ID N°6)
W-Abu-G-W-S-S-C-S-R-S (SEQ ID N°7)
L-Nal2-S-G-W-S-S-C-S-R-S (SEQ ID N°8)

Il est à noter que tous les composés peptidiques selon l'invention peuvent être obtenus par les différents modes de synthèse chimique des peptides, comme la synthèse sur support solide par exemple.

Ils peuvent aussi être obtenus par des systèmes d'expression et de constructions génétiques comportant une séquence ou un fragment d'ADN pouvant coder pour des composés peptidiques décrits selon l'invention.

D'une manière générale, ces composés peptidiques ou polypeptidiques peuvent être obtenus au moyen de tout système procaryote ou eucaryote dans lequel une séquence en acides nucléiques correspondante, apte à exprimer lesdits peptides ou polypeptides, a été introduite de manière à pouvoir recueillir son produit d'expression.

On préfère dans le cadre de la présente invention tout particulièrement des composés peptidiques ou polypeptidiques qui contiennent au plus 150 acides aminés, et encore plus préférentiellement au plus 100 acides aminés, sauf si ledit peptide ou polypeptide contient :
plusieurs fois une même séquence selon X2-G-W-S-S-X1-S-X3 (SEQ ID N°10), ou
   - différentes séquences selon X2-G-W-S-S-X1-S-X3 (SEQ ID N°10), ou
   - une séquence dérivée de celle-ci, telle que la séquence.

On peut aussi utiliser des peptides qui consistent en la séquence selon X2-G-W-S-S-X1-S-X3 (SEQ ID N°10) ou en une séquence dérivée de celle-ci.

### 2.2 Détermination de la stabilité des peptides :

Les peptides synthétiques ont été mis en solution aqueuse (1,5 mg de peptide dissout dans 1000 µL d'eau stérile).

Leur pureté a été testée par HPLC, elle était d'au moins 97,0%.

Ces solutions ont ensuite été diluées : à 0,4 mL de chacune de ces solutions, on a ajouté 1,1 mL d'eau purifiée.

Ces solutions ont été placées en étuve à 22°C et 50% d'humidité relative, et analysées après : 0, 3, 7, 11 et 22 jours.

L'analyse a été faite par HPLC en gradient de phase inverse à une concentration de 0,4 mg/mL, avec les paramètres suivants :
- cartouche Symétrie RP18 (Waters) 125 x 4,6 mm
- phases mobiles
A : H₂O + TFA 0.1 % (w/v)
B : Acétonitrile + TFA 0.1 % (v/v)
- détection : 210 nm
- débit : 1 ml/min
- volume d'injection : 20 µl de solution (soit 8 µg de peptide)
- gradient :

| | A | B |
|---|---|---|
| 0 min | 90% | 10% |
| 25 min | 40% | 60% |
| 26 min | 90% | 10% |
| 40 min | 90% | 10% |

Les échantillons ont été placés en étuve à 22 °C, 50 % d'humidité relative et analysés à : 0 jours, 3 jours, 7 jours, 11 jours et 22 jours.

Les résultats sont présentés dans le Tableau 1 et sur la Figuré 1.

Le paramètre « TR » exprime le temps de rétention du peptide en minutes dans la colonne de HPLC.

Le paramètre « Pureté » exprimé la pureté du peptide en % tel que déterminé par HPLC.

La colonne « Impuretés : TR est % » indique pour le pic supplémentaire le plus intense, le temps de rétention en minutes et le pourcentage en masse. La colonne SEQ indique le numéro de la séquence (SEQ ID N° X).

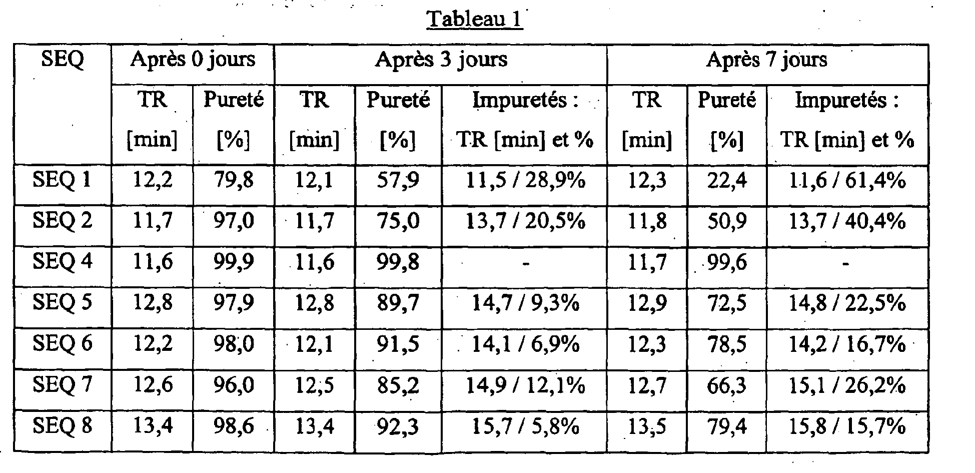

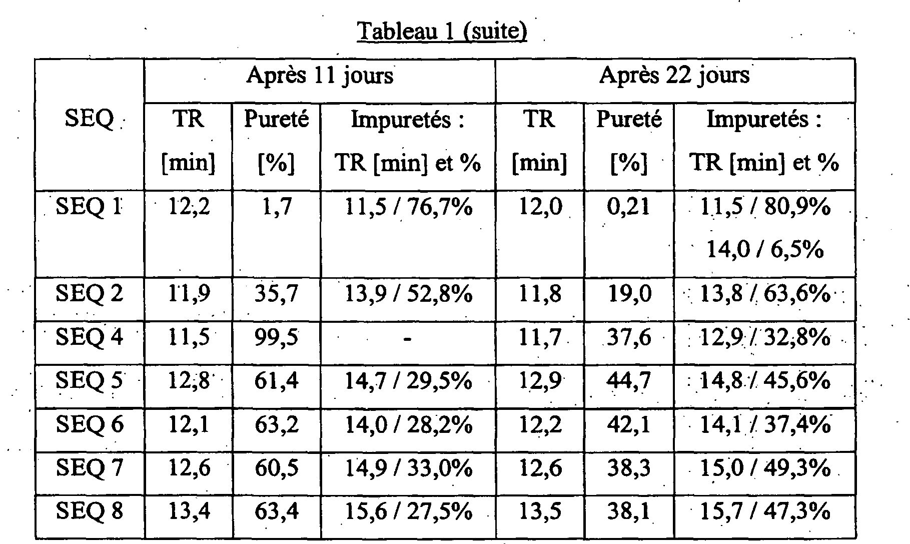

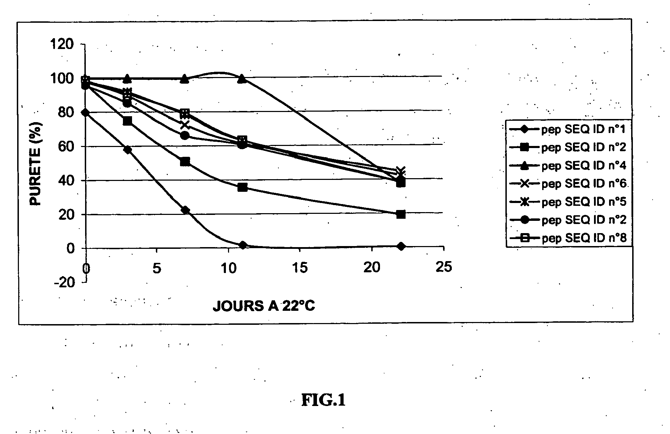

Les résultats obtenus (Fig. 1) montrent clairement que les analogues sont généralement plus stables que le peptide de (SEQ ID N°1) qui se dégrade plus vite. Cette dégradation est principalement due à un changement de conformation (cyclisation).

### 2.3 Détermination in vitro de l'activité biologique des peptides sur des cellules nerveuses de rat

L'activité biologique des différents peptides a été analysée par ajout de chacun des peptides dans des cultures de cellules nerveuses et suivi des modifications morphologiques de ces cellules.

Parmi les peptides selon l'invention, la séquence W-S-G-W-S-S-Abu-S-R-S (SEQ ID N°4) a finalement été préférée de par le remplacement de l'acide aminé Cystéine par l'acide aminé acide 2-aminobutyrique (Abu) ; ce qui permettait d'empêcher les interactions possibles entre deux peptides par formation de liaisons interchaînes. Synthétisé de façon standard sous la forme d'un peptide possédant les extrémités NH₂- et -COOH terminales, ce peptide ne s'est pas montré efficace sur l'induction de la pousse neuritique.

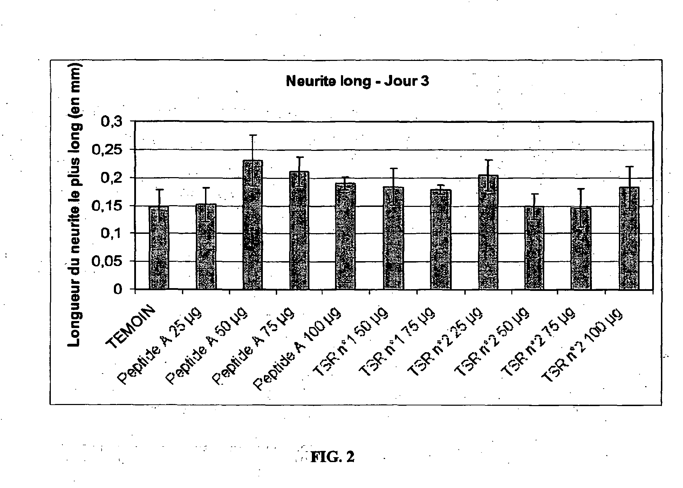

Toutefois, et de façon surprenante, l'activité d'induction de cette séquence a été recouvrée lorsque l'extrémité carboxyterminale a été remplacée par une extrémité aminée. Ainsi, le peptide de séquence _{NH2}-W-S-G-W-S-S-Abu-S-R-S-_{NH2} s'est montré actif. Il est à noter que ce remplacement de l'extrémité carboxyterminale par une extrémité aminée n'a aucune incidence sur l'effet du peptide de W-S-G-W-S-S-C-S-R-S-C-G (SEQ ID N°1). Les résultats sont portés sur la figure 2.

### a) Matériel et méthodes

Des cellules de la lignée B104, issues de neuroblastome de rat, sont cultivées à raison de 1000 cellules par puits dans des plaques de 48 puits préalablement recouverts de poly-lysine (fourni par Sigma-Aldrich), dans du milieu de Eagle modifié par Dubelco (DMEM), supplémenté de glutamine et d'antibiotique, et additionné de 10% de sérum de veau foetal pendant la phase d'adhésion. Le milieu est ensuite remplacé par un nouveau milieu DMEM ne contenant pas de sérum et permettant la différenciation des cellules. Après traitement, c'est-à-dire après ajout du composé à tester, les cellules sont maintenues en culture à 37°C sous une atmosphère à 10% de CO₂. L'évolution de la morphologie cellulaire et la prolifération sont suivies par examen microscopique en contraste de phase chaque jour sur la période de culture. La différenciation cellulaire, et en particulier l'effet sur la pousse neuritique, est alors mesurée.

### b) Effet sur adhésion cellulaire des peptides

L'adhésion des neurones à leur substrat est une étape clé dans le processus de croissance neuritique. Il est important de noter que 24 heures après l'adjonction des peptides selon l'invention, l'étalement du corps cellulaire est favorisé dans les puits traités avec les peptides alors que dans les cultures témoins, les cellules sont essentiellement fusiformes.

De plus, on peut observer que cet effet est présent aussi bien pour les cellules traitées avec le peptide W-S-G-W-S-S-C-S-R-S-C-G (SEQ ID N°1) que pour celles traitées avec le peptide W-S-G-W-S-S-C-S-R-S (SEQ ID N°2) et les peptides selon l'invention.

### c) Effet dose-réponse

Au bout de 24 heures de mise en culture, les cellules commencent à montrer l'ébauche d'un ou de deux prolongements neuronaux, indépendamment de la présence ou de l'absence des peptides.

Après 3 jours de culture, des pousses neuritiques importantes sont observées chez les cellules cultivées en présence des peptides W-S-G-W-S-S-C-S-R-S-C-G (SEQ ID N°1), W-S-G-W-S-S-C-S-R-S (SEQ ID N°2) et les peptides selon l'invention, alors que les cultures témoins ne montrent que peu de pousses neuritiques, durant cette période de culture et se présentent sous forme de fuseau caractéristique.

Pour les peptides biologiquement actifs, la réponse des cellules B 104 est proportionnelle à la concentration de peptide présente dans le milieu de culture, aussi bien pour le peptide W-S-G-W-S-S-C-S-R-S-C-G (SEQ ID N°1) de référence que pour le peptide W-S-G-W-S-S-C-S-R-S (SEQ ID N°2) et les peptides selon l'invention.

En effet, le nombre de sprouting et de neurites, ainsi que la taille du neurite le plus long par cellule augmentent avec la concentration de peptide actif.

Dans les conditions de cet essai, le peptide W-S-G-W-S (SEQ ID N°3) n'a pas d'effet biologique.

### d) Mesure du nombre de sprouting par cellule

Les cellules traitées par les peptides selon l'invention ou par les peptides W-S-G-W-S-S-C-S-R-S-C-G (SEQ ID N°1) et W-S-G-W-S-S-C-S-R-S (SEQ ID N°2), présentent une augmentation du nombre de sprouting, alors que peu de bourgeonnements d'extension neuritique sont observés dans la culture témoin.

Au bout de 3 jours de culture, les cultures témoins présentent environ un sprouting par cellule, alors que dans les cultures traitées avec le peptide W-S-G-W-S-S-C-S-R-S-C-G (SEQ ID N°1), les cellules présentent environ 1,5 sprouting en moyenne.

De même, et par comparaison, le peptide W-S-G-W-S-S-C-S-R-S (SEQ ID N°2) semble aussi actif que le peptide W-S-G-W-S-S-C-S-R-S-C-G (SEQ ID N°1).

L'effet dose-réponse est également observé avec ce peptide (Figure 3).

Par contre, le peptide W-S-G-W-S (SEQ ID N°3) semble n'avoir aucune activité d'induction lors des expérimentations effectuées.

### e) Mesure du nombre de neurites par cellule

Un effet inducteur de la pousse neuritique est observé en présence des peptides W-S-G-W-S-S-C-S-R-S-C-G (SEQ ID N°1), W-S-G-W-S-S-C-S-R-S (SEQ ID N°2) et des peptides selon l'invention, par rapport au témoin.

Aucun effet n'est observé en présence du peptide W-S-G-W-S (SEQ ID N°3).

Ainsi, au bout de 3 jours de culture, les cellules traitées présentent en moyenne plus du double de neurites par cellule par rapport aux cultures témoins (Figure 4).

### f) Mesure de la longueur de la pousse neuritique

La mesure des prolongements neuritiques montre une simulation du phénomène de pousse neuritique chez les cellules traitées par les peptides par rapport aux cellules témoins.

Ainsi, après 3 jours de culture, dans les cultures témoins, la taille du neurite le plus long est de 0,148 mm ± 0,03 mm, alors que celle obtenue dans les cultures traitées avec les peptides biologiquement actifs, tels que les peptides selon l'invention, est significativement supérieur.

Avec le peptide W-S-G-W-S-S-C-S-R-S-C-G (SEQ ID N°1), la taille du neurite le plus long est de 0,181 mm ± 0,02 mm, et elle est de 0,196 mm ± 0,028 mm dans les cultures traitées avec le peptide W-S-G-W-S-S-C-S-R-S (SEQ ID N°2) (Figure. 5).

### 2.4 Activité in vivo

Une première caractérisation *in vivo* des phénomènes de repousse neuritique et de survie neuronale a été effectuée sur deux modèles d'animaux différents dans le cas du trauma de la moelle épinière (réparation de la moelle épinière après rupture).

Ainsi, dans le cas de la souris avec lésion ischémique partielle entraînant une paralysie, qui peut avoir une évolution positive spontanée, on a observé une activité régénératrice du peptide W-S-G-W-S-S-C-S-R-S-C-G (SEQ ID N°1) et des composés peptidiques selon l'invention (évaluation fonctionnelle au niveau de la marche notamment).

Dans le cas de rats avec lésion au niveau de la moelle épinière (lésion provoquée par aspiration de la matière grise), on a observé qu'un tube de collagène rempli de peptide placé dans la cavité a montré une repousse des fibres nerveuses dans le tube (évaluation immnunohistochimique).

Ces résultats montrent que les composés peptidiques ou polypeptidiques selon l'invention peuvent être utilisés pour la préparation d'un médicament destiné à :
- stimuler la repousse neuritique, ou
- améliorer la survie neuronale en cas de trauma de moelle épinière, ou
- être utilisé en tant que principe actif d'un tel médicament, seul ou en combinaison avec d'autres principes actifs.

Par ailleurs, les composés peptidiques selon l'invention peuvent être utilisés pour la préparation d'un médicament destiné à améliorer les symptôme ou à guérir des maladies dans lesquelles certains neurones (tels que les neurones cholinergiques, dopaminergique et motoneurones) sont atteints ou disparaissent, telles que la maladie de Parkinson et l'amyotrophie latérale spinale, ou en tant que principe actif d'un tel médicament, seul ou en combinaison avec d'autres principes actifs.

Sans être lié par cette théorie, les inventeurs pensent qu'un des effets des composés peptidiques selon l'invention est de permettre le rétablissement des contacts synaptiques entre neurones en cours de destruction.

En ce qui concerne le dosage des composés peptidiques selon l'invention, l'étude de leur effet sur des cellules O-2A (lignée cellulaire oligodendrocytaire) de rat en culture primaire montre que les indications sont différentes selon la quantité de peptide utilisée. A titre d'exemples :
- à une dose de l'ordre de 25µg, le peptide selon l'invention doit plutôt être employé pour étudier des phénomènes de prolifération,
- à une dose de l'ordre de 50µg, le peptide selon l'invention a un effet de survie meilleur qu'avec le cocktail de facteurs de croissance utilisé classiquement. Cependant, il conserve aussi un effet sur la prolifération des cellules. Pharmacologiquement, une dose de l'ordre de 50 µg convient notamment dans des cas de manipulation *ex vivo* de cellules, en vue de leur transplantation par exemple.

- à une dose d'environ 75 ou 100µg, le peptide selon l'invention induit plutôt la survie, voire même la différenciation des neurones.
- il convient de noter qu'à une dose d'environ 150 µg, certains peptides selon l'invention montrent un effet létal (phénomène non observé avec les neurones).

D'autres renseignements sur le dosage du peptide selon l'invention découlent des essais *in vitro*.

Nous décrivons ici un autre essai *in vivo* pour démontrer l'effet biologique des composés peptidiques selon l'invention. Cet essai concerne un modèle de lésion de la moelle épinière par contusion; il mime ce qu'on observe chez l'homme lorsque la moelle épinière est endommagée lors d'un accident de la route par exemple. Les rats ont été anesthésiés par une injection intrapéritonéale d'un mélange de Kétamine et de Xylazine à respectivement des concentrations de 80 et 10 mg/kg. La vessie des rats était systématiquement vidée avant la procédure chirurgicale. Le dos a été alors rasé et désinfecté soigneusement. Une laminectomie a été alors réalisée en T9-T10, le périoste était alors retiré et moelle épinière fixée et stabilisée en utilisant les clamps solidaires de l'impacteur dit "NYU device" qui permet une lésion contrôlée au moyen d'une masselotte de 10 g lâchée d'une hauteur réglée à 25 mm sur le site de lésion. La lésion ainsi engendrée a été considérée comme plutôt sévère et a entraîné une paralysie de la partie basse des animaux (pattes arrières, contrôle de la vessie, ...). Le composé peptidique selon l'invention a été administré aux animaux au moyen d'un petit cube d'environ 2 mm³ de Surgicoll (Collin) saturé de 30 µl du composé à teste, qui était alors placé au niveau du site de lésion. Les plans musculaires et cutanés ont été refermés et les animaux placés sous une lampe chauffante jusqu'à leur réveil. L'efficacité du traitement a été évaluée en utilisant le test du BBB, échelle sensorimotrice utilisée dans les laboratoires travaillant sur les lésions de la moelle épinière mais également en vérifiant le bon retour du contrôle vésicale durant toute la phase post-opératoire. Ainsi, en qui concerne la locomotion, aucune différence significative n'a pu être dégagée entre les animaux traités avec le peptide selon l'invention aux doses utilisées et le groupe control. En revanche, l'analyse du timing de récupération de la fonction urinaire autonome et de la taille de la vessie au cours du temps indique la présence de différences significatives (p<0,05) entre les profils des groupes traités et le groupe témoin. Cela montre que les animaux traités avec les composés peptidiques selon l'invention récupèrent plus rapidement une fonction vésicale autonome. Les résultats sont montrés sur les figures 6 et 7 (composé peptidique NS selon l'invention, SEQ ID NO 4).

### 2.5 Expériences in vitro pour extrapolation in vivo

Ces expériences ont eu pour objectif de pouvoir préciser les conditions d'injection des composés peptidiques melon l'invention chez l'animal lors des tests de validation de l'activité *in vivo.* Ainsi, il est important de savoir le moment d'injection, la quantité et le mode d'administration. Il est également important de savoir si une injection unique est suffisante ou s'il doit être injecté à plusieurs reprises.

### a) Plusieurs injections versus une seule injection

On voit une augmentation du nombre de cellules avec un seul ajout de peptide (comme précédemment décrit), cet effet est amplifié avec 3 additions quotidiennes du composé peptidique selon l'invention.

Cette amplification de l'effet est également observée pour la différenciation (taille du neurite) avec des ajouts répétés.

Ces résultats sont plutôt en faveur d'une injection répétée du composé peptidique selon l'invention.

### b) Retrait de peptide toutes les 24 heures

Au niveau de la survie et/ou prolifération, on trouve le profil attendu avec une légère augmentation du nombre de cellules en présence de peptide (10%). Avec le retrait, on voit la différence à partir de 24 heures seulement et pas 1 heure. Selon ces résultats, la fenêtre d'action du peptide est donc comprise entre 1 heure et 24 heures.

### 2.6 Autres effets biologiques des composés peptidiques selon l'invention

### a) Croissance d'oligodendrocytes

Des expériences sur une autre population cellulaire d'origine animale, les oligodendrocytes, ont montré qu'à une concentration particulière, les composés peptidiques selon l'invention ont aussi un rôle sur ces cellules ; ils stimulent la survie et favorisent la différenciation.

Cette action sur ce type de cellule est intéressante à plusieurs titres et notamment dans les cas de rupture de la moelle épinière (celle-ci entraîne une démyélinisation qu'il faudra combler lors du phénomène de réparation).

D'autre part, cela ouvre d'autres perspectives d'applications potentielles, par exemple pour les maladies de dégénérescence touchant d'autres cellules que les neurones, comme la sclérose en plaque. La sclérose en plaques attaque en effet les cellules de synthèse de myéline, les oligodendrocytes. Elle se caractérise aussi par un vaste phénomène inflammatoire, avec une activation massive des astrocytes. Les composés peptidiques selon l'invention pourraient donc intervenir en complément d'une thérapie anti-inflammatoire.

Un premier essai a été décrit ci-dessus sous point 2.4. Nous décrivons ici un autre essai en détail :

Des oligodendrocytes, obtenus après dissection, ont été mis en culture à raison de 50000 cellules par puits dans des plaques multipuits de 24 puits dans 500 µl de milieu DMEM + 10% de sérum de voeu foetal. Après 16h d'adhésion le milieu a été remplacé par 480 µl de milieu Neurobasal + compléments N2 (Invitrogen). Un composé peptidique selon l'invention où son véhicule (eau) a été ajouté à raison de 20 µl par puits. Les cultures ont été placées à 37°C sous une atmosphère de 5% de CO₂. Au bout de 72 h de cultures la moitié du milieu de culture par puit a été changée et complétée par une nouvelle solution de traitement (milieu + peptide ou son véhicule). Les cultures ont été maintenues à 37°C sous une atmosphère de 5% de CO₂. Les cultures ont été régulièrement observées et la densité cellulaire ainsi que leur morphologie ont été évalués. Les résultats indiquent que le composé peptidique selon l'invention favorise la survie de ces cellules ainsi que la pousse de leurs extensions.

### b) Croissance de cellules nerveuses humaines

Des cellules de la lignée SY5Y, issues d'un neuroblastome humain, ont été cultivées dans du milieu de Eagle modifié par Dubelco (DMEM) supplémenté de 10% de sérum de veau fatal (SVF), puis différenciées en flasque en présence d'acide rétinoïque (ATRA, Sigma) 20 µM pendant 6 jours. Les flasques ont ensuite été trypsinées, et les cellules ensemencées dans du DMEM + 10% SVF en plaque multipuits le temps de l'adhésion puis le sérum est complètement retiré. Les cellules ont été traitées par le composé peptidique selon l'invention.

On a observé que dans ces conditions, les cellules SY5Y émettent des prolongements de manière plus importante dans les conditions traitées avec le peptide stabilisé par rapport à la condition contrôle en absence de celui-ci. Un réseau dense d'extension nerveuses s'est formé dans les puits, indiquant que le peptide selon l'invention est capable de stimuler la croissance des extensions nerveuses et induire la formation d'un réseau neuritique important.

### c) Activité neuroprotectrice

Les composés peptidiques exercent également une activité neuroprotectrice, qui peut être mise en évidence par exemple en traitant une culture de SY5Y avec un agent de stress, tel que :
- l'eau oxygénée (H₂O₂) qui induit un stress oxydatif et entraine la mort des cellules ;
- le glutamate, un acide aminé excitateur, qui est un agent convulsivant et un agent qui participe au processus de progression des dommages (extension de la zone de lésion) causés lors d'un traumatisme nerveux;
- le peptide beta amyloide, qui est à l'origine de la formation des plaques séniles causant les lésions observées dans la maladie d'Alzheimer et est une des cibles pour combattre et traiter cette maladie.

### d) Dégénérescence axonale

L'activité d'un composé peptidique selon l'invention est testée chez l'animal dans un modèle de dégénérescence de la moelle épinière et de dégénérescence axonale en particulier. Il s'appuie sur l'utilisation du modèle animal Plp-/-, relevant de la pathologie humaine SPG2 (formes légères de paraplégies spastiques). Une première étape d'injections intrathécales de peptide NS (SEQ ID NO 4) à une dose de 37.5µg/kg à des animaux normaux contrôles durant 5 mois (de 2 mois à 7 mois) a été réalisée pour tester la toxicité potentielle des injections répétées (n=20). Les résultats ont démontré l'innocuité du produit administré de façon chronique, et ont ainsi permis de poursuivre les expérimentations. La stratégie de traitement a consisté en l'administration du peptide chez des souris PLP-/- âgées de 6 mois, c'est-à-dire avant que la dégénérescence axonale ne commence. Les effets du traitement sont comparés, à un groupe contrôle qui sera traité avec le véhicule de suspension du peptide seulement et l'efficacité du principe actif est mesurée selon diverses méthodes d'analyse incluant des tests comportementaux qui permettront de suivre les capacités sensori-motrice et l'activité locomotrice des différents groupes d'animaux (système Rotarod), ainsi que des analyses neuropathologiques.

### 2.7 Utilisation des composés peptidiques selon l'invention

Les composés peptidiques, selon l'invention peuvent être utilisés de différentes manières.

Ils peuvent être utilisés dans une composition pharmaceutique ou pour la réalisation d'un médicament. Dans ces compositions ou médicaments, l'agent bioactif peut se présenter sous des formes diverses de compositions, à savoir sous forme de solutions, en général aqueuses, ou sous forme lyophilisée, ou sous forme de gel ou d'hydrogel, ou sous forme d'émulsion ou bien toute autre forme pharmaceutiquement et physiologiquement acceptable. Ces médicaments ou compositions sont notamment destinés au traitement des maladies neurodégénératives et / ou à la régénération de la moelle épinière ou des nerfs périphériques après traumatismes. Leur utilisation dans la régénération des cellules du système nerveux peut se faire soit par application directe à un patient, soit de manière extracorporelle.

Les composés peptidiques selon l'invention peuvent aussi être utilisés comme auditif pour les cultures cellulaires de cellules nerveuses. Un tel additif comporte au moins un des composés peptidiques selon l'invention. Cet additif peut être utilisé dans un support pour la culture de cellules nerveuses. Un support de culture selon l'invention comporte un corps de base, de forme généralement creuse, et un revêtement sur lequel les cellules peuvent se fixer. Le corps de base peut être une plaque à puits. Le revêtement comporte des additifs actifs, dont au moins un des composés peptidiques selon l'invention.

## Revendications

1. Composé peptidique contenant au moins la séquence en acides aminés suivantes :
X2-G-W-S-S-X1-S-X3 (SEQ ID N°10)
dans lequel :
X1 est C ou Abu ;
X2 est W- S ou W - Abu ou L - Nal2 - S ;
X3 est R - S, R - Abu ou K-(iPr)-S ;
ledit composé étant **caractérisé en ce que** la séquence (SEQ ID N°10) est sélectionnée dans le groupe constitué par les séquences :
W-S-G-W-S-S-Abu-S-R-S (SEQIDN°4)
W-S-G-W-S-S-C-S-R-Abu (SEQ ID N°5)
W-S-G-W-S-S-C-S-K-(iPr)-S (SEQIDN°6)
W-Abu-G-W-S-S-C-S-R-S (SEQIDN°7)
L-Nal2-S-G-W-S-S-C-S-R-S (SEQ ID N°8).

2. Composé peptidique selon la revendication 1, comportant la séquence (SEQ ID N°4) sous la forme _{NH2}-W-S-G-W-S-S-Abu-S-R-S_{-NH2}.

3. Composition pharmaceutique comprenant à titre de principe actif au moins un composé peptidique selon l'une quelconque des revendications 1 ou 2.

4. Utilisation extracorporelle de composés peptidiques selon l'une quelconque des revendications 1 ou 2 dans la régénération des cellules du système nerveux.

5. Utilisation de composés peptidiques selon l'une quelconque des revendications 1 ou 2 pour la réalisation de médicaments destinés au traitement des maladies neurodégénératives.

6. Utilisation de composés peptidiques selon l'une quelconque des revendications 1 ou 2 pour la réalisation de médicaments destinés à la régénération de la moelle épinière ou des nerfs périphériques après traumatismes.

7. Additif pour les cultures cellulaires de cellules nerveuses, **caractérisé en ce qu'**il comporte au moins un composé peptidique selon l'une quelconque des revendications 1 ou 2.

8. Support pour la culture de cellules nerveuses, **caractérisé en ce qu'**il comporte l'additif selon la revendication 7.

## Claims

1. Peptide compound comprising at least the following amino acid sequence :
X2-G-W-S-S-X1-S-X3 (SEQ ID N°10)
wherein :
X1 is C or Abu ;
X2 is W- S or W - Abu or L - Nal2 - S ;
X3 is R - S, R - Abu or K-(iPr)-S ;
said compound being **characterized in that** the sequence (SEQ ID N°10) is selected from the group formed by the sequences :
W-S-G-W-S-S-Abu-S-R-S (SEQIDN°4)
W-S-G-W-S-S-C-S-R-Abu (SEQ ID N°5)
W-S-G-W-S-S-C-S-K-(iPr)-S (SEQ ID N°6) W-Abu-G-W-S-S-C-S-R-S (SEQIDN°7)
L-Nal2-S-G-W-S-S-C-S-R-S (SEQ ID N°8).

2. Peptide compound according to claim 1, comprising the sequence (SEQ ID N°4) in the form _{NH2-}W-S-G-W-S-S-Abu-S-R-S_{-NH2}.

3. Pharmaceutical composition comprising as an active principle at least one peptide compound according to any of claims 1 or 2.

4. Extracorporeal use of peptide compounds according to any of claims 1 or 2 for cell regeneration in the nervous system.

5. Use of peptide compounds according to any of claims 1 or 2 for the preparation of drugs for the treatment of neurodegenerative diseases.

6. Use of peptide compounds according to any of claims 1 or 2 for the preparation of drugs for the regeneration of the spinal cord or peripheral nerves after traumatic damage.

7. Additive for neuronal cell cultures, **characterized in that** it comprises at least one peptide compound according to any of claims 1 or 2.

8. Substrate for neuronal cell cultures, **characterized in that** it comprises the additive according to claim 7.

## Patentansprüche

1. Peptidverbindung, die wenigstens einer der folgenden Aminosäuresequenzen enthält :
X2-G-W-S-S-X1-S-X3 (SEQIDN°10)
worin :
X1 is C or Abu ;
X2 ist W- S oder W - Abu oder L - Nal2 - S ;
X3 ist R - S, R - Abu oder K-(iPr)-S ;
**dadurch** charakterisiert, dass die Sequenz aus der Gruppe ausgewählt ist, die aus den folgenden Sequenzen gebildet wird :
W-S-G-W-S-S-Abu-S-R-S (SEQIDN°4)
W-S-G-W-S-S-C-S-R-Abu (SEQ ID N°5)
W-S-G-W-S-S-C-S-K-(iPr)-S (SEQ ID N°6)
W-Abu-G-W-S-S-C-S-R-S (SEQIDN°7)
L-Nal2-S-G-W-S-S-C-S-R-S (SEQ ID N°8).

2. Peptidverbindung nach Anspruch 1, die die Sequenz (SEQ ID N°4) in der Form _{NH2-}W-S-G-W-S-S-Abu-S-R-S_{-NH2} enhält.

3. Pharmakologisch wirksame Zusammensetzung, die als aktives Prinzip wenigstens eine Peptidverbindung nach Anspruch 1 oder 2 enthält.

4. Extrakorporelle Verwendung von Peptidverbindungen nach Anspruch 1 oder 2 zur Regenerierung von Zellen des Nervensystems.

5. Verwendung von Peptidverbindungen nach Anspruch 1 oder 2 zur Herstellung von Medikamenten zur Behandlung neurodegenerativer Erkrankungen.

6. Verwendung von Peptidverbindungen nach Anpruch 1 oder 2 zur Herstellung von Medikamenten zur Regenerierung des Rückenmarks oder peripherer Nerven nach traumatischer Beschädigung.

7. Additiv für Nervenzellkulturen, **dadurch** charakterisiert dass es wenigstens eine Peptidverbindung nach einem der Ansprüche 1 oder 2 enthält.

8. Substrat für Nervenzellkulturen, **dadurch** charakterisiert, dass es das Additiv nach Anspruch 7 enthält.
